# FASCICULE DE BREVET EUROPEEN

(11) **EP 2 501 377 B1**
(45) Date de publication et mention de la délivrance du brevet: **27.01.2016**
(21) Numéro de dépôt: 10778669.1
(22) Date de dépôt: 19.11.2010
(51) Int. Cl.: A61K 31/34, A61K 31/426, A61K 31/4418, A61K 31/505, A61K 31/513, A61K 31/52, A61K 31/536, A61K 31/661, A61K 31/675, A61K 31/7068, A61P 31/18

(54) **NOUVEAUX SCHEMAS D'ADMINISTRATION DE MULTITHERAPIES UTILES POUR LE TRAITEMENT DES PERSONNES ATTEINTES DU VIRUS DE L'IMMUNODEFICIENCE HUMAINE (VIH)**
Neue Verabreichungsschemata von Multitherapieformen, die zur Behandlung von HIV-positiven Personen geeignet sind
New multi-therapy administration patterns suitable for treating persons affected by human immunodeficiency virus (HIV)

(30) Priorité: 20.11.2009 EP 09176666
(43) Date de publication de la demande: 26.09.2012
(73) Titulaire: Assistance Publique Hôpitaux De Paris, 75001 Paris (FR); L'UNIVERSITE DE VERSAILLES SAINT-QUENTIN-EN- YVELINES, 78035 Versailles Cedex (FR)
(72) Inventeur: LEIBOWITCH, Jacques, F-75006 Paris (FR)
(74) Mandataire: Icosa
(86) Numéro de dépôt international: PCT/EP2010/067851
(87) Numéro de publication internationale: WO 2011/061302

(56) Documents cités:
- COHEN C J ET AL: "Pilot study of a novel short cycle antiretroviral treatment interruption strategy: 48-week results of the five-days-on, two-days-off (FOTO) study", 20070101, vol. 8, no. 1, 1 janvier 2007 (2007-01-01) , pages 19-23, XP009133720, cité dans la demande

## Description

La présente invention a pour objet de nouveaux schémas d'administration de multithérapies utiles pour le traitement des personnes affectées par le Virus de l'Immunodéficience Humaine (VIH), responsable du Syndrome d'ImmunoDéficience Acquise (SIDA).

Le Virus de l'Immunodéficience Humaine (ou VIH) est un rétrovirus du genre des lentivirus, c'est-à-dire un virus avec une longue période d'incubation, ce qui implique une évolution lente de la maladie.

Comme tous les virus, le VIH est incapable de se multiplier tout seul. Il doit préalablement pénétrer dans une cellule et en prendre le contrôle. Les cellules cibles du VIH sont celles présentant des récepteurs CD4 à leur surface. Ainsi, les lymphocytes TCD4+, les macrophages, les cellules dendritiques et les cellules microgliales cérébrales peuvent être infectées par le VIH.

Lorsque le VIH infecte une cellule cible, il en prend contrôle. Alors, le virus commence à fabriquer de nouvelles copies de lui-même : c'est la phase de reproduction ou de réplication. Les virions ainsi produits infectent d'autres cellules. Faute de traitement, les experts estiment que le VIH peut fabriquer jusqu'à 10 milliards de copies virales chaque jour.

Deux sérotypes du VIH ont été identifiés à ce jour, le VIH-1, qui est présent dans la plupart des pays du monde, et le VIH-2 qui est principalement présent en Afrique de l'Ouest.

Il est communément admis que la réplication du virus se déroule en plusieurs étapes majeures :
1- Fixation ou attachement à une cellule cible
2- Fusion, pénétration et décapsidation
3- Transcription inverse
   Cette étape est spécifique aux rétrovirus : en effet, ces derniers ayant pour génome de l'ARN et non de l'ADN, une opération de transcription, "convertissant" l'ARN viral en ADN viral, seul à pouvoir être intégré dans le génome de la cellule cible, est nécessaire. Cette transcription est réalisée par l'enzyme de transcriptase inverse (TI).
4- Intégration
   L'ADN bicaténaire ainsi formé, étroitement associé à l'intégrase et d'autres composants protéiques viraux et cellulaires dans un complexe appelé complexe de pré-intégration, pénètre dans le noyau cellulaire. L'ADN s'intègre ensuite au hasard dans le génome de la cellule cible, sous l'effet de l'enzyme intégrase.
5- Formation d'un ARN messager (ARNm)
6- Epissage de l'ARNm ainsi obtenu
7- Traduction de l'ARNm
8- Maturation
9- Assemblage
   Les protéines de structure du virus (matrice, capside et nucléocapside) sont produites sous forme de polyprotéines. A la suite de l'étape de maturation, les différentes protéines sont liées entre elles et sont transportées vers la membrane de la cellule cible où elles rejoignent les glycoprotéines virales membranaires. Des ARN viraux rejoignent les protéines virales. Les protéines de structure s'assemblent pour former la capside (enveloppe de nature protéique recouvrant l'ADN ou l'ARN, l'ensemble étant désigné par nucléocapside) et la matrice, englobant cet ensemble.
10- Bourgeonnement
   La capside sort de la cellule infectée.
11- Maturation des virus
   Une protéase virale coupe les liens qui unissent les différentes protéines de structure (matrice, capside et nucléocapside). Suite à ces coupures, les virions (particules virales complètes avec leur enveloppe protéinique externe (capside) et leurs molécules d'ARN ou d'ADN à l'intérieur) deviennent alors infectieux et sont prêts à infecter de nouvelles cellules.

Une fois la séropositivité établie, le suivi régulier du patient est mis en place. Deux facteurs principaux sont habituellement surveillés afin de suivre l'évolution de la maladie :

### 1- Le taux de lymphocytes TCD4+

Le taux de lymphocytes TCD4+ est utilisé pour suivre la progression de l'infection vers le déficit immunitaire occasionné par le VIH. La numération des lymphocytes TCD4+ correspond au nombre de cellules T4 présentes dans le sang. Un taux normal chez l'Homme se situe entre 500 et 1 500 TCD4+/mm3 de sang. Il a été généralement admis que :
- jusqu'à 500 TCD4+/mm³ de sang, le patient pouvait vivre dans des conditions normales sans nécessiter de traitement ;
- à partir de 350 TCD4+/mm³ de sang, une proposition de traitement antiviral se discute, le résultat attendu étant le contrôle de l'activité de reproduction du VIH, et, secondairement, une remontée au moins partielle du taux de TCD4+ ;
- en dessous de 200 TCD4+/mm³ de sang, le patient est considéré comme immunodéprimé, courant le risque de souffrir de maladies définissant un SIDA avéré. Le traitement antiviral avec ou sans antibioprophylaxie s'impose comme seul capable d'éviter ces complications.

### 2- La charge virale

La concentration des particules virales HIV dans un volume de sang donne une estimation objective du nombre total de virions fraîchement produits par l'organisme du sujet infecté. La mesure est faite selon des techniques standardisées peu variables d'un laboratoire à l'autre s'il utilise ces techniques validées. Le résultat est donné en log10 du nombre de copies/ml. L'erreur sur la quantification (nombre de copies du virus) est telle qu'une variation inférieure ou égale à 0,5 est dite non significative.

La différence entre deux mesures de charge virale espacées dans le temps permet d'évaluer le taux de reproduction du VIH et donc l'évolution de l'infection. Il est généralement admis qu'il existe des liens entre la charge virale et le niveau du déficit immunitaire, manifesté par la disparition des lymphocytes TCD4+.

La charge virale est, à la date de la présente invention, le meilleur indicateur de l'évolution du virus chez le patient. L'état des connaissances permet d'avancer en outre qu'un patient dont la charge virale plasmatique est inférieure à 50 copies/ml peut être considéré comme « non transmetteur d'infection» par voie muqueuse.

A la date de la présente invention, aucune composition pharmaceutique ne permet d'éradiquer définitivement le VIH chez une personne ayant contracté le virus, mais certaines compositions permettent de réprimer la réplication du VIH, un contrôle attesté par le maintien d'une charge virale constamment inférieure à 50 copies/ml de plasma. Ce contrôle permet d'arrêter la progression de la maladie vers un SIDA, et assure une espérance de vie pour le porteur de VIH correctement traité proche ou égale de celle des personnes de même âge et de même sexe.

Depuis le début des années 1980, de nombreuses recherches ont aboutit à l'identification d'un grand nombre d'antirétroviraux ayant pour fonction d'interférer et de bloquer les différents mécanismes nécessaire à la réplication du virus VIH, en ciblant plus particulièrement tel ou tel enzyme du VIH nécessaire à sa réplication ou en affectant les mécanismes physico chimiques présidant à l'entrée du virus dans la cellule cible.

A la date de la présente invention, les antirétroviraux constituent les seuls médicaments utilisés utilement contre le VIH. L'objectif premier et principal de cette thérapie, notamment chez le patient naïf de tout traitement, est de maintenir la charge virale en dessous du seuil de détection de 50 copies/ml de plasma aussi longtemps que possible, faute de quoi la thérapie antivirale risque de perdre son efficacité dans la durée, du fait de l'émergence de virus résistants aux drogues antivirales administrées (Hammer SM, Saag MS, Schechter M, et al., Treatment for adult HIV infection: 2006 recommandations of the International AIDS Society-USA panel. Top HIV Med (2006) 14:827-43)

Les médicaments anti VIH sont regroupés dans quatre grandes classes d'antirétroviraux, différant par leur mode d'action sur le virus HIV et contre sa reproduction et ou sa propagation dans l'organisme du porteur :
On distingue tout d'abord les inhibiteurs de la transcriptase inverse, qui inhibent le recopiage de l'ARN viral en ADN proviral, première étape dans la réplication du virus à partir de l'ARN viral. Dans cette classe, on distingue :
   - les inhibiteurs nucléosidiques ou nucléotidiques de la transcriptase inverse (INTI) ; et
   - les inhibiteurs non nucléosidiques (INNTI)

Les INTI correspondent à la première classe d'antirétroviraux mis sur le marché. Comme exemples de composés INTI, on peut citer la zidovudine (AZT, *Retrovir*®) et la stavudine (d4T, *Zerit*®) (deux analogues de la thymidine), la didanosine (ddI, *Videx*®), l'abacavir (ABC, *Ziagen*®) et le tenofovir (TDF, *Viread*®) (trois analogues de l'adénosine), et la lamivudine (3TC, *Epivir*®) et l'emtricitabine (FTC, *Emtriva*®) (deux analogues de la cytosine).

Les INNTI sont des inhibiteurs sélectifs et puissants de la transcriptase inverse du VIH. Comme exemples de composés INNTI on peut citer la nevirapine (NVP, *Viramune*), l'etravirine (ETV, *Intelence*®), et l'efavirenz (EFV, *Satstiva*®)*.* Ils ne sont actifs que sur les VIH-1.

On distingue ensuite des inhibiteurs de la protéase HIV (IP) qui agissent en inhibant l'action de l'enzyme qui dirige le découpage exact des protéines virales précurseurs de structures nécessaires à la formation du matériel HIV infectieux, et notamment les virions HIV, matériels capables de se propager dans l'organisme pour infecter de nouvelles cellules permissives. Sous l'action des inhibiteurs de la protéase HIV, on obtient des pseudos virions incapables d'infecter de nouvelles cellules. Comme exemples de composés IP, on peut citer, dans l'ordre historique de leur mise sur le marché, saquinavir (SQV, *Invirase*®), ritonavir (RTV, *Norvir*®), indinavir (IDV, *Crixivan*®), amprenavir (APV, *Agenerase*®), nelfinavir (NFV, *Viracept*®), atazanavir (ATZ, *Reyataz*®), fosamprenavir (FPV, *Telzir*®), tipranavir (TPV, *Aptivus*®), et darunavir (DRV, *Prezista*®).
Chacune de ces IP a pour propriété pharmacocinétique de s'éliminer de l'organisme du patient avec rapidité par la voie des cytochromes P450 ; le blocage partiel de cette voie d'élimination par un produit comme le ritonavir, inhibiteur puissant des fonctions cytochrome P450, prolonge sensiblement la durée de vie pharmaceutique de l'IP prescrite. Le ritonavir donné à doses faibles « booste » l'anti protéase HIV administrée dans le même temps au patient, dans la mesure où il en augmente les taux dans le sang, et en prolonge la demi-vie utile dans l'organisme.

On distingue encore les inhibiteurs d'intégrase qui bloquent l'action d'un enzyme du VIH dont la fonction élective est d'élaguer les extrémités des ADN proviraux HIV de façon à rendre cet ADN apte à servir de matrice à la transcription de l'ADN proviral en ARN HIV. Les inhibiteurs de l'intégrase rendent cet enzyme momentanément inapte à sa fonction d'élagage ADN, empêchant par là la reproduction du génome viral dans sa cellule cible. Comme exemples de composés inhibiteurs d'intégrase on peut citer le raltégravir et l'elvitégravir (GS 9137).

On distingue enfin les inhibiteurs de fusion-lyse qui interviennent avant le début du cycle biochimique de réplication du VIH, en bloquant la marche infectieuse du VIH au niveau de certaines protéines présentes à la surface des virions, ou en interférant avec les capacités de liaisons de ces protéines de surface avec des co-récepteurs présents eux-mêmes en surface de cellules cibles du VIH. Comme exemples de composés inhibiteurs de fusion-lyse, on peut citer l'enfuvirtide (*Fuzeon*®) et le maraviroc (*Celsentri*®)*.*

Administrés seuls, la plupart des agents antirétroviraux se sont montrés seulement partiellement efficaces, incapables le plus souvent de bloquer suffisamment la reproduction du VIH pour obtenir une réduction optimale de la charge virale ou empêcher sa remontée

Pour palier à cette déficience, de nombreuses multithérapies, et en particulier des trithérapies, ont été mises au point au fil des années.

La trithérapie consiste en la co-administration de trois agents antirétroviraux, se présentant soit sous la forme de trois médicaments distincts administrés séparément, soit sous la forme d'une forme pharmaceutique unitaire contenant les trois principes actifs.

Grâce à ces multithérapies, et en particulier aux trithérapies utilisées depuis 1996, le taux de mortalité dû au SIDA a été réduit de façon significative.

Sur la base de leur efficacité démontrée, et de leur acceptabilité, les combinaisons antirétrovirales préférées pour le démarrage d'une thérapie anti-VIH chez les patients vierges de traitement antérieurs ont pour socle des combinaisons de deux INTI associées soit à un IP boosté par le ritonavir, soit un INNTI (Gazzard B. British HIV Association (BHIVA) guidelines for the treatment of HIV-infected adults with antiretroviral therapy (2006). HIV Med (2006) 7:487-503).

Exceptionnellement un troisième inhibiteur de la transcriptase inverse est ajouté à la combinaison constituée d'un couple de nucléosides et d'un INNTI pour former une quadrithérapie, mais celles-ci, de même que les trithérapies associant trois INTI n'ont généralement pas été validées.

Parmi les trithérapies disponibles à la date de la présente invention, on peut citer les trithérapies associant :
- une paire d'INTI choisie parmi :
   o lamivudine ou emtricitabine, et zidovudine ;
   o lamivudine ou emtricitabine, et stavudine (cependant ces deux derniers couples de nucléosides analogues sont plus souvent délaissés par les prescripteurs en Occident en raison de leurs effets métaboliques indésirables) ;
   o lamivudine ou emtricitabine et abacavir;
   o lamivudine ou emtricitabine et tenofovir ; ou
   o lamivudine ou emtricitabine et didanosine ;
- avec la combinaison du ritonavir avec un IP choisi parmi lopinavir, fosamprenavir, atazanavir et darunavir ; ou avec un INNTI choisi parmi nevirapine, efavirenz et etravirine

Cependant, nombre des trithérapies disponibles à la date de la présente invention sont marquées d'échappements virologiques, c'est-à-dire une charge virale chez le patient supérieure à 100 copies/ml de plasma mesurée lors deux dosages consécutifs rapprochés; le taux des «fuites virales» s'élevant avec les années de prises ininterrompues. Dans ces cas, le taux d'échappements s'élève à 10% et plus des patients traités au bout de seulement 48 semaines de traitement, et peut passer les 20% voire les 30% au terme de 3 ou 4 années de traitements ininterrompus. Ces échappements sont la marque de combinaisons antivirales sous-optimales, et mettent en avant autant de situations dans lesquelles peut survenir une sélection de virus HIV portant des mutations de résistance au moins partielle aux composants médicamenteux de la combinaison (First-line antiretroviral therapy with efavirenz or lopinavir/ritonavir plus two nucleoside analogues: the SUSKA study, a non-randomized comparison from the VACH cohort, Pere Domingo et al., Journal of Antimicrobial Chemotherapy (2008) 61, 1348-1358). C'est le cas en particulier de la plupart des trithérapies associant seulement trois composants inhibiteurs de la transcriptase inverse et des trithérapies combinant deux composants inhibiteurs de la transcriptase inverse à la nevirapine. (*Risk of Early Virological Failure of Once-Daily Tenofovir-Emtricitabine plus Twice-Daily Nevirapine in Antiretroviral Therapy-Naive HIV-Infected Patients,* Giuseppe Lapadula, Silvia Costarelli, Eugenia Quiros-Roldan, et al, Clinical Infectious Diseases 2008, 46:1127-1129 ; et *High rate of early virological failure with the once-daily tenofovir* /*lamivudine* /*nevirapine combination in naive HIV-1-infected patients-authors' response,* D. Rey, B. Hoen, P. Chavanet, et al, J. Antimicrob. Chemother 2009; 63: 1080-1081).

D'autre part, de nombreux effets secondaires indésirables sont associés à l'utilisation de ces médicaments, parmi lesquels les lipodystrophies ou répartitions anormales des graisses solides dans le corps, en relation principalement, sinon exclusivement, aux combinaisons antivirales impliquant un inhibiteur nucléosidique de la famille des analogues de thymidine tel que la stavudine (d4T) ou la zidovudine (AZT) ; l'acidose lactique se caractérisant par une respiration profonde et rapide, une somnolence, des nausées, des vomissements et/ou des douleurs d'estomac ; des sensations de vertiges ; des difficultés à dormir ; des difficultés à se concentrer ; des rêves anormaux ; des éruptions cutanées ; des inflammations ou des infections diverses ; et/ou des problèmes osseux...

Le nombre de trithérapies disponibles à la date de la présente invention permet d'administrer à chaque patient affecté par le VIH différentes compositions au cours de son traitement afin d'adapter au mieux ledit traitement à l'évolution de l'infection et à la tolérance par le patient. Mais la nécessité d'une administration journalière sept jours par semaine fait de ces thérapies des traitements lourds et contraignants pour le patient, et tend à augmenter l'intensité des effets secondaires ressentis par celui-ci.

A la date de la présente invention, deux trithérapies (Trizivir® un médicament mis sur le marché par le laboratoire pharmaceutique Glaxo Smith Kline et Atripla®, un médicament mis sur le marché par le laboratoire pharmaceutique Gilead) permettent l'administration journalière sept jours par semaine du traitement sous la forme d'une forme posologique unitaire.
Trizivir® se présente sous la forme d'un unique comprimé pelliculé comprenant :
- 150 mg de lamivudine ;
- 300 mg de zidovudine ; et
- 300 mg d'abacavir base (351 mg de sulfate d'abacavir).
Atripla® se présente sous la forme d'un unique comprimé pelliculé comprenant :
- 600 mg d' efavirenz ;
- 200 mg d'emtricitabine ; et
- 245 mg de tenofovir disoproxil fumarate (exprimé en tenofovir disoproxil).

Cette seconde composition pharmaceutique, qui est parmi les trithérapies les plus efficaces actuellement présentes sur le marché, nécessite néanmoins une administration journalière sept jours par semaine, ce qui est loin de favoriser la meilleure observance du traitement par les patients.

De plus, ni Atripla®, ni Trizivir® n'ont permis une diminution des effets secondaires mentionnés ci-dessus.

Enfin, le coût par patient et par an des multithérapies existantes à la date de la présente invention demeure excessivement élevé. Ainsi, à titre d'exemple, Atripla® est vendu en France sous forme flacon contenant 30 comprimés (soit un mois de traitement) pour le prix de 834,30€, soit un coût annuel par patient d'environ 10.000€. Or, si les traitements actuels permettent de fortement limiter le développement du virus VIH chez les patients atteints, ils ne permettent en aucun cas de l'éradiquer. Le coût du traitement des personnes affectées par le VIH peut donc atteindre des montants très importants, amenés à augmenter fortement dans le futur.

En 2007, une étude isolée a tenté de démontrer qu'il était possible de diminuer à cinq jours le nombre d'administration hebdomadaire de différentes trithérapies existantes *(Pilot Study of a Novel Short*-*Cycle Antiretroviral Treatment Interruption Strategy: 48-Week Results of the Five-Days-On, Two-Days-Off (FOTO) Study,* Calvin J. Cohen, MD, Amy E. Colson, Alexander G. Sheble-Hall, et al, HIV Clin Trials 2007;8(1):19-23). Dans cette étude conduite sur trente patients dont le virus HIV est contrôlé durablement par différentes trithérapies ininterrompues, le régime de traitement hebdomadaire a été réduit à cinq jours par semaine (et deux jours d'arrêt). A la 24^{ème} et 48^{ème} semaine de ce traitement, le virus est resté sous contrôle chez 26 de 29 patients (89.6%). Cependant, de l'aveu même des auteurs, les bénéfices entrevus dans le cadre de l'étude « FOTO » sont encore très incertains et de telles régimes posologiques ne devraient pas être utilisés avant que ces résultats ne soient confirmés dans le cadre d'une étude plus large. De plus,ce document ne donne aucune indication quant à la possibilité d'une éventuelle réduction supplémentaire du nombre de prises hebdomadaires des trithérapies existantes. Surtout, il insiste sur le fait qu'il faut réserver les traitements intermittents aux seules trithérapies comportant un agent anti-rétroviral non nucléosidique, tel qu'efavirenz ou nevirapine, de longue durée de vie naturelle dans l'organisme, excluant de fait les combinaisons comprenant des agents ayant un temps de demi-vie court dans le plasma tels que les anti-protéases (IP).

Ces travaux demeurent en outre isolés et, à la date de la présente invention, la plupart des spécialistes s'accordent à considérer qu'une diminution du nombre d'administrations hebdomadaires des trithérapies existantes ne manquerait pas d'augmenter le nombre d'échappement virologiques chez les patients traités. Ainsi une diminution du nombre d'administrations hebdomadaires des trithérapies existantes est généralement associée à un échec thérapeutique certain. A titre d'exemple, le Professeur Delfraissy considère la non-observance du traitement comme la principale cause de l'échec thérapeutique (« Prise en charge thérapeutique des personnes infectées par le VIH - Rapport 2004 - Sous la direction du Professer Jean-français Delfraissy », 2004, Éditions Flammarion, p. 48-49).

De plus, une étude publiée postérieurement à l'étude « FOTO » (*Relationship between Adherence Level, Type of the Antiretroviral Regimen*, *and Plasma HIV Type 1 RNA Viral Load: A Prospective Cohort Study,* M. Martin, E. Del Cacho, C. Codina, et al, AIDS Research and Human Retroviruses, October 2008, 24(10): 1263-1268. doi:10.1089/aid.2008.0141) résume bien le préjugé dominant selon lequel réduire la quantité des agents antiviraux chez un patient doit entraîner la reprise de la réplication du VIH, à proportion inverse de la pression exercée quotidiennement par la trithérapie en question. Ainsi, en comparaison avec des patients observant le traitement prescrit à plus de 90%, cette étude fait état d'un risque d'échappement virologique :
- 9 fois plus grand chez les patients n'observant le traitement qu'à 80 à 89,9%, soit, pour une trithérapie supposant une administration journalière sept jours par semaine, pour les patients prenant leur traitement environ six jours sur sept ;
- 45,6 fois plus grand chez les patients n'observant le traitement qu'à 70 à 79,9%, soit, pour une trithérapie supposant une administration journalière sept jours par semaine, pour les patients prenant leur traitement environ cinq à six jours sur sept ; et
- 77,3 fois plus grand chez les patients n'observant le traitement à moins de 70%, soit, pour une trithérapie supposant une administration journalière sept jours par semaine, pour les patients prenant leur traitement moins de cinq jours sur sept.

D'autre part, une autre étude également publiée postérieurement à l'étude « FOTO » (*Not all missed doses are the same: sustained NNRTI treatment interruption predict HIV rebound at low-to-moderate adherence levels,* Parienti JJ, Das-Douglas M, Massari V, Guzman D, Deeks SG, Verdon R, Bangsberg D.R., PLoS One, July 30, 2008; 3(7):e2783) enseigne que toute interruption du traitement supérieure à 2 jours augmente les risques de « rebond » virologique, c'est-à-dire les risques d'une reprise de la réplication du VIH.

Il a pourtant été découvert, de façon totalement inattendue, que certaines multithérapies pouvaient être administrées au patient selon un schéma d'administration différent de celui recommandé et utilisé dans le cadre du traitement du VIH à la date de la présente invention, permettant une nette diminution du nombre d'administrations hebdomadaires du traitement, sans pour autant affecter ou diminuer l'efficacité de ce dernier.

La présente invention a donc pour objet une composition pharmaceutique pour le traitement du virus de l'immunodéficience humaine (VIH) chez l'être humain comprenant trois ou quatre principes actifs choisis comme étant :
- un inhibiteur nucléosidique de la transcriptase inverse (INTI) choisi parmi la lamivudine et l'emtricitabine ;
- un inhibiteur nucléosidique ou nucléotidique de la transcriptase inverse (INTI) choisi parmi la didanosine, l'abacavir et le tenofovir ; et
- la combinaison du ritonavir avec un inhibiteur de la protéase (IP) choisi parmi le lopinavir, le fosamprenavir, l'atazanavir et le darunavir ; ou un inhibiteur non nucléosidique de la transcriptase inverse (INNTI) choisi parmi l'efavirenz et l'etravirine ;
pour une administration journalière un à quatre jours par semaine audit être humain.

La composition pharmaceutique selon l'invention permet de diminuer le nombre d'administrations hebdomadaires au patient, tout en maintenant une efficacité au moins comparable à celle de la trithérapie considérée lorsqu'elle est administrée une fois par jour et sept jours par semaine audit patient.

De plus, dans le cadre de la présente invention, il peut bien entendu être envisagé d'administrer au patient affecté par le VIH une seule des compositions selon l'invention pendant toute la durée de son traitement ou, au contraire, d'administrer successivement plusieurs des compositions de l'invention au cours dudit traitement, afin d'adapter au mieux le traitement à l'évolution de la maladie. A titre d'exemple, un patient affecté par le VIH peut ainsi être traité initialement par une trithérapie associant l'emtricitabine (FTC), le tenofovir (TDF) et l'efavirenz (EFV), puis par une trithérapie associant l'emtricitabine (FTC), le tenofovir (TDF) et l'atazanavir (ATZ) « boosté » par le ritonavir (r) ; puis par une trithérapie associant l'emtricitabine (FTC), le tenofovir (TDF) et la lopinavir (LPV) « boostée » par le ritonavir (r) ; puis par une trithérapie associant l'emtricitabine (FTC), le tenofovir (TDF) et le darunavir (DRV) « boosté » par le ritonavir (r).

Dans le cadre de la présente invention :
- VIH désigne exclusivement le VIH-1;
- on entend par « sel pharmaceutiquement acceptable » d'un principe actif tout sel d'addition dudit principe actif avec un acide minéral ou organique par action d'un tel acide au sein d'un solvant organique ou aqueux tel qu'un alcool, une cétone, un éther ou un solvant chloré, et qui soit acceptable d'un point de vue pharmaceutique ;
- on entend par « dérivé pharmaceutiquement acceptable » d'un principe actif tout «prodrogue» ou « métabolite » dudit principe actif, ainsi que leur sel pharmaceutiquement acceptable ;
- on entend par « prodrogue » d'un principe actif tout composé dont la biotransformation dans l'organisme aboutit audit principe actif ;
- on entend par « métabolite » d'un principe actif tout produit intermédiaire résultant de la transformation dudit principe actif dans l'organisme lors d'un processus métabolique ;
- on entend par « administration journalière » une administration une fois par jour ou une administration une fois par 24 heures ;
- on entend par « calendrier continu » le traitement thérapeutique continu d'un patient, comprenant l'administration successive d'une ou plusieurs compositions thérapeutiques (dont multithérapies, selon l'invention ou non), identiques ou différentes, chacune avec son propre schéma d'administration thérapeutique (nombre d'administrations journalières et nombre de jours d'administration sur une période donnée, semaine par exemple) et ce, de façon illimitée et non séquencée ou espacée dans le temps, c'est-à-dire sans interruption de traitement ;
- la lamivudine (ou 3TC) désigne la (2*R*,5*S*)-(-)-4-amino-1-[2-(hydroxymethyl)-1,3-oxathiolan-5-yl]-1H-pyrimidin-2-one, ainsi que ses sels ou dérivés s pharmaceutiquement acceptables ;
- l'emtricitabine (ou FTC) désigne la L-2',3'-didéoxy-5-fluoro-3'-thiacytidine, ainsi que ses sels ou dérivés pharmaceutiquement acceptables ;
- la didanosine (ou DDI) désigne la L 2',3'-didéoxyinosine, ainsi que ses sels ou dérivés pharmaceutiquement acceptables ;
- l'abacavir (ou ABC) désigne le [(1*S*,4*R*)-4-[2-amino-6-(cyclopropylamino)-9H-purin-9-yl]cyclopent-2-ényl]méthanol, ainsi que ses sels ou dérivés pharmaceutiquement acceptables, parmi lesquels l'abacavir sulfate ;
- le tenofovir (ou TDF) désigne le L (R)-9-(2-phosphonylméthoxy-propyl)adénine, ainsi que ses sels ou dérivés pharmaceutiquement acceptables, parmi lesquels le tenofovir disoproxil ou le fumarate de tenofovir disoproxil.
- l'efavirenz (ou EFV) désigne la (S)-6-chloro-4-(cyclopropylethynyl) -1,4-dihydro-4-(trifluoromethyl) -2H-3,1-benzoxazin-2-one, ainsi que ses sels ou dérivés pharmaceutiquement acceptables ;
- l'etravirine (ou ETV) désigne le 4-({6-amino-5-bromo-2 -[(4-cyanophenyl)amino]pyrimidin-4-yl}oxy) -3,5-dimethylbenzonitrile, ainsi que ses sels ou dérivés pharmaceutiquement acceptables ;
- le ritonavir désigne le 1,3-thiazol-5-ylmethyl N-[(2S,3S,5S)-3-hydroxy-5-[(2S)-3-methyl-2-{[methyl({[2-(propan-2-yl)-1,3-thiazol-4-yl]methyl})carbamoyl]amino}butanamido]-1,6-diphenylhexan-2-yl]carbamate, ainsi que ses sels ou dérivés pharmaceutiquement acceptables ;
- le lopinavir (ou ABT-378) désigne le (2S)-N-[(2S,4S,5S)-5-[2-(2,6-dimethylphenoxy)acetamido]-4-hydroxy-1,6-diphenylhexan-2-yl]-3-methyl-2-(2-oxo-1,3-diazinan-1-yl)butanamide, ainsi que ses sels ou dérivés pharmaceutiquement acceptables ;
- le fosamprenavir (ou TZV) désigne le {[(2R,3S)-1-[N-(2-methylpropyl)(4-aminobenzene)sulfonamido]-3-({[(3S)-oxolan-3-yloxy]carbonyl}amino)-4-phenylbutan-2-yl]oxy}phosphonic acid, ainsi que ses sels ou dérivés pharmaceutiquement acceptables, parmi lesquels le fosamprenavir calcique;
- l'atazanavir (ou ATZ) désigne le methyl N-[(1S)-1-{[(2S,3S)-3-hydroxy-4-[(2S)-2-[(methoxycarbonyl)amino]-3,3-dimethyl-N'-{[4-(pyridin-2-yl)phenyl]methyl}butanehydrazido]-1-phenylbutan-2-yl]carbamoyl}-2,2-dimethylpropyl]carbamate, ainsi que ses sels ou dérivés pharmaceutiquement acceptables, parmi lesquels l'atazanavir calcique; et
- le darunavir désigne le [(1R,5S,6R)-2,8-dioxabicyclo[3.3.0]oct-6-yl] N-[(2S,3R)-4-[(4-aminophenyl)sulfonyl- (2-methylpropyl)amino]-3-hydroxy-1-phenyl- butan-2-yl] carbamate, ainsi que ses sels ou dérivés pharmaceutiquement acceptables, parmi lesquels le darunavir ethanolate.

Préférentiellement, la présente invention a pour objet une composition pharmaceutique telle que définie ci-dessus, dans laquelle les caractéristiques suivantes sont choisies seules, ou en combinaison :
- le « premier » INTI est choisi comme étant l'emtricitabine ;
- le « second » INTI est choisi comme étant le tenofovir ou la didanosine, de préférence encore le second INTI est choisi comme étant le tenofovir ;
- l'IP est choisi comme étant le darunavir ou le atazanavir, de préférence encore l'IP est choisi comme étant le darunavir ; et/ou
- l'INNTI est choisi comme étant l'efavirenz.

De façon toute à fait préférée, la présente invention a pour objet une composition pharmaceutique comprenant, à titre de principes actifs, l'emtricitabine, le tenofovir et l'efavirenz.

Comme autres exemples de compositions pharmaceutiques selon la présente invention, on peut notamment citer les compositions pharmaceutiques comprenant :
- l'emtricitabine, la didanosine et la combinaison du ritonavir avec le lopinavir;
- l'emtricitabine, la didanosine et la combinaison du ritonavir avec le fosamprenavir ;
- l'emtricitabine, la didanosine et la combinaison du ritonavir avec l'atazanavir ;
- l'emtricitabine, la didanosine et la combinaison du ritonavir avec le darunavir ;
- l'emtricitabine, la didanosine et l'efavirenz ;
- l'emtricitabine, la didanosine et l'etravirine ;
- la lamivudine, la didanosine et la combinaison du ritonavir avec le lopinavir;
- la lamivudine, la didanosine et la combinaison du ritonavir avec le fosamprenavir ;
- la lamivudine, la didanosine et la combinaison du ritonavir avec l'atazanavir ;
- la lamivudine, la didanosine et la combinaison du ritonavir avec le darunavir ;
- la lamivudine, la didanosine et l'efavirenz ;
- la lamivudine, la didanosine et l'etravirine ;
- l'emtricitabine, l'abacavir et la combinaison du ritonavir avec le lopinavir;
- l'emtricitabine, l'abacavir et la combinaison du ritonavir avec le fosamprenavir ;
- l'emtricitabine, l'abacavir et la combinaison du ritonavir avec l'atazanavir ;
- l'emtricitabine, l'abacavir et la combinaison du ritonavir avec le darunavir ;
- l'emtricitabine, l'abacavir et l'efavirenz ;
- l'emtricitabine, l'abacavir et l'etravirine ;
- la lamivudine, l'abacavir et la combinaison du ritonavir avec le lopinavir;
- la lamivudine, l'abacavir et la combinaison du ritonavir avec le fosamprenavir ;
- la lamivudine, l'abacavir et la combinaison du ritonavir avec l'atazanavir ;
- la lamivudine, l'abacavir et la combinaison du ritonavir avec le darunavir ;
- la lamivudine, l'abacavir et l'efavirenz ;
- la lamivudine, l'abacavir et l'etravirine ;
- l'emtricitabine, le tenofovir et la combinaison du ritonavir avec le lopinavir;
- l'emtricitabine, le tenofovir et la combinaison du ritonavir avec le fosamprenavir ;
- l'emtricitabine, le tenofovir et la combinaison du ritonavir avec l'atazanavir ;
- l'emtricitabine, le tenofovir et la combinaison du ritonavir avec le darunavir ;
- l'emtricitabine, le tenofovir et l'etravirine ;
- la lamivudine, le tenofovir et la combinaison du ritonavir avec le lopinavir;
- la lamivudine, le tenofovir et la combinaison du ritonavir avec le fosamprenavir ;
- la lamivudine, le tenofovir et la combinaison du ritonavir avec l'atazanavir ;
- la lamivudine, le tenofovir et la combinaison du ritonavir avec le darunavir ;
- la lamivudine, le tenofovir et l'efavirenz ; et
- la lamivudine, le tenofovir et l'etravirine ;

La composition pharmaceutique selon la présente invention contient les principes actifs en quantité suffisante pour assurer l'effet thérapeutique souhaité, c'est-à-dire le traitement du VIH en maintenant chez le patient traité une charge virale inférieure à 50 copies/ml, de préférence inférieure ou égale à 20 copies/ml.

Le cas échéant, la composition pharmaceutique selon la présente invention permet également le maintien ou la remontée du taux de lymphocytes TCD4+ à un niveau de préférence supérieur aux taux de TCD4+/mm³ du patient avant traitement effectif.

De préférence, les quantités d'agents antirétroviraux utilisées pour préparer la composition pharmaceutique selon l'invention sont les suivantes :
- de 200 à 400 mg de lamivudine ;
- de 100 à 300 mg d'emtricitabine ;
- de 150 à 350 mg de didanosine ;
- de 500 à 700 mg d'abacavir ;
- 145 à 345 mg de tenofovir ;
- de 100 à 200 mg de ritonavir ;
- de 400 à 800 mg de lopinavir ;
- de 600 à 1400 mg de fosamprenavir ;
- de 200 à 400 mg d'atazanavir ;
- de 600 à 1200 mg de darunavir;
- 100 à 700 mg d'efavirenz ;
- 300 à 500 mg d'etravirine.

De préférence encore, les quantités d'agents antirétroviraux utilisées pour préparer la composition pharmaceutique selon l'invention sont identiques à celles classiquement administrées de façon journalière sept jours par semaine au patient suivant les trithérapies connues à la date de la présente invention, soit:
- 300 mg de lamivudine ;
- 200 mg d'emtricitabine ;
- 250 mg de didanosine ;
- 600 mg d'abacavir ;
- 245 mg de tenofovir ;
- 100 mg de ritonavir ;
- 600 mg de lopinavir ;
- 1200 mg de fosamprenavir ;
- 300 ou 400 mg d'atazanavir ;
- 800 ou 900 mg de darunavir ;
- 200, 400 ou 600 mg d'efavirenz ;
- 400 mg d'etravirine.

La composition pharmaceutique selon la présente invention peut être formulée sous toute forme galénique nécessaire à son administration. En particulier, s'agissant d'administration par voie orale, les compositions selon la présente invention peuvent être formulées sous forme de comprimés enrobés ou non, effervescents, solubles, orodispersibles, gastrorésistants ou à libération modifiée ; de dragées ; de capsules à enveloppe dure (ou gélules) ; de capsules à enveloppe molle ; de granules ; de granulés ; de pilules ; de pastilles. S'agissant d'administration par voie systémique, la composition selon l'invention peut être formulée sous forme de poudre lyophilisée stérile pour injection. Les compositions pharmaceutiques selon la présente invention pourront donc comprendre, en plus des principes actifs, tout adjuvant de formulation pharmaceutiquement acceptable, connu de l'homme du métier et qui est nécessaire à la préparation de la composition pharmaceutique sous la forme souhaitée.

La composition pharmaceutique selon l'invention peut être administrée de façon journalière un jour, deux jours, trois jours ou quatre jours par semaine au patient, tout en maintenant une efficacité au moins comparable à celle de la trithérapie considérée lorsqu'elle est administrée de façon journalière sept jours par semaine audit patient. De préférence, la présente invention a pour objet une composition pharmaceutique telle que définie précédemment pour une administration journalière deux à quatre jours par semaine, de préférence encore trois ou quatre jours par semaine

La composition pharmaceutique selon l'invention peut être administrée à tout moment de la journée, avant, pendant ou après les repas, sans que cela n'influe sur l'efficacité du traitement.

La composition pharmaceutique selon l'invention peut être administrée selon un calendrier continu.

La composition pharmaceutique selon l'invention peut être administrée à tout patient affecté par le VIH. Néanmoins, on préférera administrer la composition selon la présente invention à un patient présentant une charge virale inférieure ou égale à 50 copies/ml de plasma depuis au moins une mesure, quel que soit le moyen thérapeutique ayant servi à atteindre un tel niveau de la charge virale.

Les trois ou quatre principes actifs constituant la composition pharmaceutique selon l'invention peuvent être administrés sous la forme d'une composition pharmaceutique unitaire comprenant les trois ou quatre principes actifs permettant une administration de ladite composition au patient en une seule prise. Une administration séparée d'un ou plusieurs des principes actifs constitutifs de composition pharmaceutique selon l'invention peut également être envisagée. Ainsi, la présente invention a également pour objet un produit pharmaceutique contenant :
- un inhibiteur nucléosidique de la transcriptase inverse (INTI) choisi parmi la lamivudine et l'emtricitabine ;
- un autre inhibiteur nucléosidique ou nucléotidique de la transcriptase inverse (INTI) choisi parmi la didanosine, l'abacavir et le tenofovir ; et
- la combinaison du ritonavir avec un inhibiteur de la protéase (IP) choisi parmi le lopinavir, le fosamprenavir, l'atazanavir et le darunavir ; ou un inhibiteur non nucléosidique de la transcriptase inverse (INNTI) choisi parmi l'efavirenz et l'etravirine ;
comme produit de combinaison pour une administration journalière simultanée, séparée ou étalée dans le temps, un à quatre jours par semaine, dans le cadre du traitement du VIH chez l'être humain.

Le produit pharmaceutique selon l'invention peut bien entendu être administré selon l'un des schémas d'administration définis précédemment.

A titre d'exemple, le produit pharmaceutique selon la présente invention peut se présenter sous la forme :
- d'une forme posologique unitaire contenant un INTI tel que défini précédemment, d'une forme posologique unitaire contenant l'autre INTI tel que défini précédemment, d'une forme posologique unitaire contenant le ritonavir et d'une forme posologique unitaire contenant l'IP tel que défini précédemment; ou
- d'une forme posologique unitaire contenant un INTI tel que défini précédemment, d'une forme posologique unitaire contenant l'autre INTI tel que défini précédemment et d'une forme posologique unitaire la combinaison du ritonavir avec un IP tel que défini précédemment; ou
- d'une forme posologique unitaire contenant un INTI tel que défini précédemment et d'une forme posologique unitaire contenant l'autre INTI et la combinaison du ritonavir avec un IP tels que définis précédemment; ou
- d'une forme posologique unitaire contenant les deux INTI tels que définis précédemment, d'une forme posologique unitaire contenant le ritonavir et d'une forme posologique unitaire contenant l'IP tel que défini précédemment ; ou
- d'une forme posologique unitaire contenant les deux INTI tels que définis précédemment et d'une forme posologique unitaire la combinaison du ritonavir avec un IP tel que défini précédemment; ou
- d'une forme posologique unitaire contenant un INTI tel que défini précédemment, d'une forme posologique unitaire contenant l'autre INTI tel que défini précédemment, d'une forme posologique unitaire contenant l'INNTI tel que défini précédemment;
- d'une forme posologique unitaire contenant un INTI tel que défini précédemment et d'une forme posologique unitaire contenant l'autre INTI et l'INNTI tels que définis précédemment; ou
- d'une forme posologique unitaire contenant les deux INTI tels que définis précédemment et d'une forme posologique unitaire contenant l'INNTI tel que défini précédemment; ou

Un autre objet de la présente invention concerne également l'utilisation d'une composition pharmaceutique telle que définie précédemment pour la préparation d'un médicament destiné au traitement du VIH chez l'être humain, ladite composition étant administrée de façon journalière un à quatre jours par semaine, de préférence deux à quatre jours par semaine, de préférence encore trois ou quatre jours par semaine audit être humain, l'administration pouvant ou non s'effectuer selon un calendrier continu.

La présente invention a également pour objet une méthode de traitement du VIH chez un être humain affecté par ce virus par l'administration journalière un à quatre jours par semaine, de préférence deux à quatre jours par semaine, de préférence encore trois ou quatre jours par semaine, d'une composition pharmaceutique telle que de définie précédemment, l'administration pouvant ou non s'effectuer selon un calendrier continu.

La présente invention est illustrée de manière non limitative par les exemples suivants.

Dans les exemples suivants (exemples 1 à 15), les doses journalières de principe actif utilisées pour traiter les patients correspondent, sauf mention contraire, aux doses suivantes :
- 300 mg de lamivudine ;
- 200 mg d'emtricitabine ;
- 250 mg de didanosine ;
- 600 mg d'abacavir ;
- 245 mg de tenofovir ;
- 100 mg de ritonavir ;
- 400 ou 600 mg de lopinavir ;
- 1200 mg de fosamprenavir ;
- 300 ou 400 mg d'atazanavir ;
- 900 mg de darunavir ;
- 600 mg d' efavirenz ;
- 400 mg d'etravirine.

### Exemple 1

Un patient affecté par le VIH a été traité selon le protocole suivant :
- traitement par une quadrithérapie;
- puis un traitement par une trithérapie associant l'emtricitabine (FTC), le tenofovir (TDF) et le lopinavir (LPV) « boosté » par le ritonavir (r), administrée de façon journalière 4 jours par semaine ;
- et enfin un traitement par une trithérapie associant l'emtricitabine (FTC), le tenofovir (TDF) et l'atazanavir (ATZ) « boosté » par le ritonavir (r), administrée de façon journalière 4 jours par semaine, puis 3 jours par semaine, puis 2 jours par semaine.

Durant ce traitement, la charge virale plasmatique, ainsi que le taux de TCD4+ dans le sang ont été mesurés.

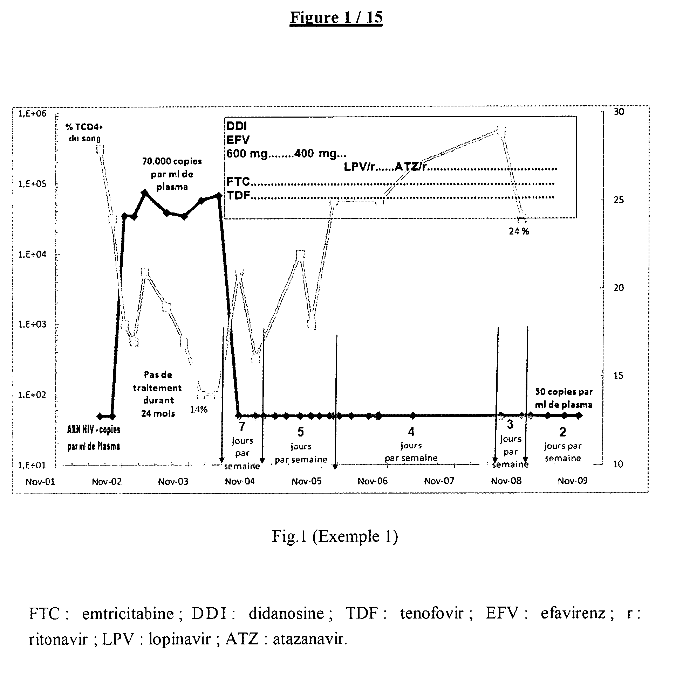

Les résultats sont rapportés dans le Figure 1.

Durant toute la période du traitement, la charge virale plasmatique du patient est demeurée inférieure ou égale à 50 copies/ml de plasma sans qu'aucun échappement virologique ne soit observé. De plus, une remontée du taux de TCD4+ dans le sang a également été observée.

### Exemple 2

Un patient affecté par le VIH a été traité selon le protocole suivant :
- traitement par une quadrithérapie;
- puis un traitement par une trithérapie associant l'emtricitabine (FTC), le tenofovir (TDF) et l'efavirenz (EFV) administrée de façon journalière 5 jours par semaine;
- et enfin un traitement par une trithérapie associant l'emtricitabine (FTC), le tenofovir (TDF) et le lopinavir (LPV) « boosté » par le ritonavir (r) (400 mg pour 65 kg), administrée de façon journalière 4 jours par semaine, puis 3 jours par semaine.

Durant ce traitement, la charge virale plasmatique, ainsi que le taux de TCD4+ dans le sang ont été mesurés.

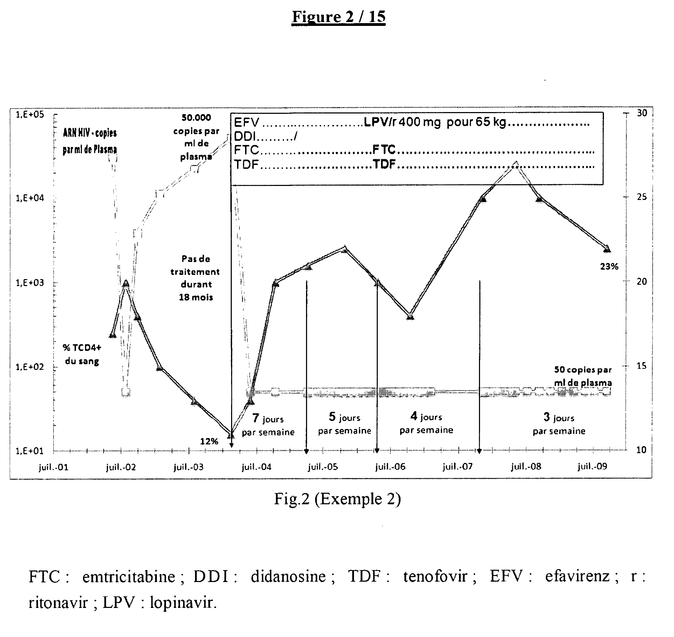

Les résultats sont rapportés dans le Figure 2.

Durant toute la période du traitement, la charge virale plasmatique du patient est demeurée inférieure ou égale à 50 copies/ml de plasma sans qu'aucun échappement virologique ne soit observé. De plus, une remontée du taux de TCD4+ dans le sang a également été observée.

### Exemple 3

Un patient affecté par le VIH a été traité selon le protocole suivant :
- traitement par plusieurs quadrithérapies;
- puis un traitement par une trithérapie associant l'emtricitabine (FTC), le tenofovir (TDF) et l'efavirenz (EFV) administrée de façon journalière 7 jours par semaine, puis 5 jours par semaine, puis 4 jours par semaine, puis 3 jours par semaine, puis 2 jours par semaine.

Durant ce traitement, la charge virale plasmatique, ainsi que le taux de TCD4+ dans le sang ont été mesurés.

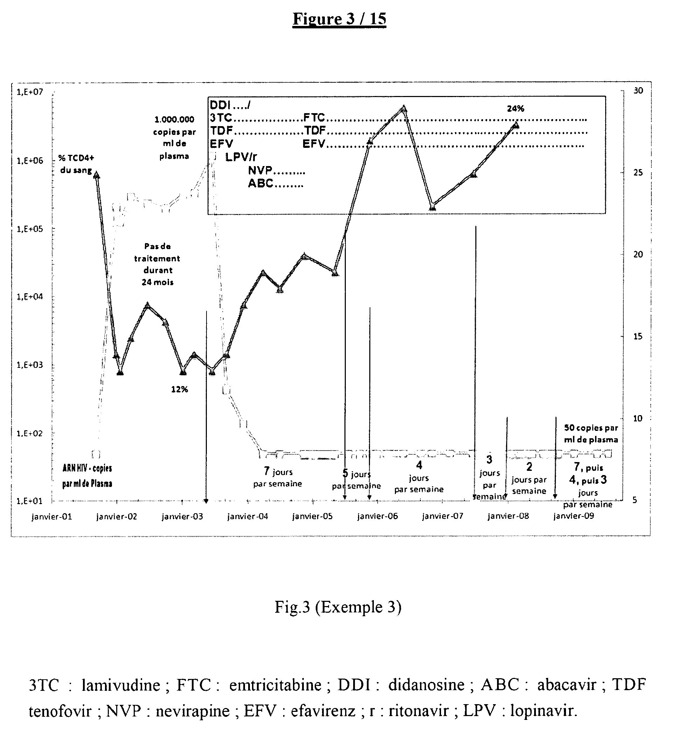

Les résultats sont rapportés dans le Figure 3.

Durant toute la période du traitement, la charge virale plasmatique du patient est demeurée inférieure ou égale à 50 copies/ml de plasma sans qu'aucun échappement virologique ne soit observé. De plus, une remontée du taux de TCD4+ dans le sang a également été observée.

### Exemple 4

Un patient affecté par le VIH a été traité selon le protocole suivant :
- traitement par une quadrithérapie;
- puis un traitement par une trithérapie associant l'emtricitabine (FTC), le tenofovir (TDF) et l'efavirenz (EFV) administrée de façon journalière 5 jours par semaine;
- et enfin un traitement par une trithérapie associant l'emtricitabine (FTC), le tenofovir (TDF) et l'atazanavir (ATZ) « boostée » par le ritonavir (r), administrée 4 jours par semaine, puis 3 jours par semaine.

Durant ce traitement, la charge virale plasmatique, ainsi que le taux de TCD4+ dans le sang ont été mesurés.

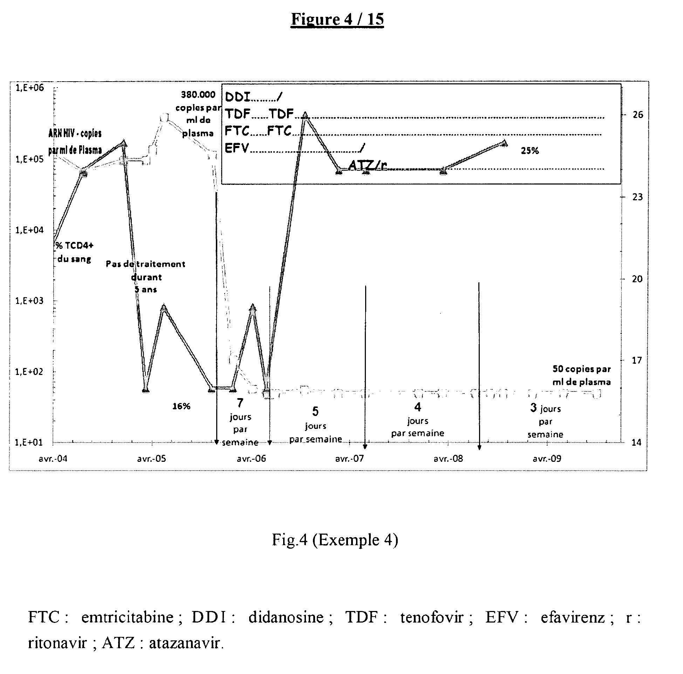

Les résultats sont rapportés dans le Figure 4.

Durant toute la période du traitement, la charge virale plasmatique du patient est demeurée inférieure ou égale à 50 copies/ml de plasma sans qu'aucun échappement virologique ne soit observé. De plus, une remontée du taux de TCD4+ dans le sang a également été observée.

### Exemple 5

Un patient affecté par le VIH a été traité selon le protocole suivant :
- traitement par une quadrithérapie;
- puis un traitement par une trithérapie associant l'emtricitabine (FTC), le tenofovir (TDF) et l'atazanavir (ATZ) « boostée » par le ritonavir (r), administrée de façon journalière 5 jours par semaine, puis 4 jours par semaine;
- et enfin un traitement par une trithérapie associant l'emtricitabine (FTC), le tenofovir (TDF) et le lopinavir (LPV) « boosté » par le ritonavir (r) 400 mg, administrée de façon journalière 3 jours par semaine.

Durant ce traitement, la charge virale plasmatique, ainsi que le taux de TCD4+ dans le sang ont été mesurés.

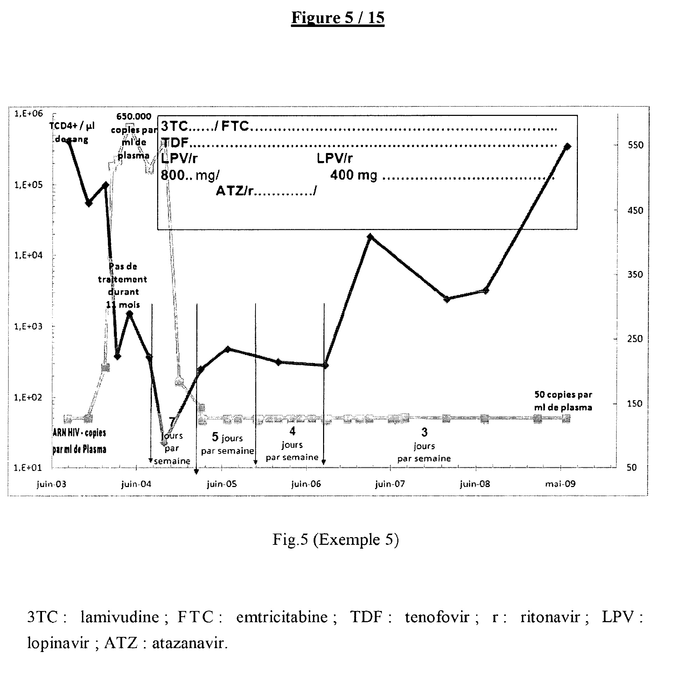

Les résultats sont rapportés dans le Figure 5.

Durant toute la période du traitement, la charge virale plasmatique du patient est demeurée inférieure ou égale à 50 copies/ml de plasma sans qu'aucun échappement virologique ne soit observé. De plus, une remontée du taux de TCD4+ dans le sang a également été observée.

### Exemple 6

Un patient affecté par le VIH non traité durant 10 mois a été traité selon le protocole suivant :
- traitement par une trithérapie associant l'emtricitabine (FTC), le tenofovir (TDF) et l'efavirenz 400 mg administrée de façon journalière 7 jours par semaine;
- puis un traitement par une trithérapie associant l'emtricitabine (FTC), le tenofovir (TDF) et l'atazanavir (ATZ) « boostée » par le ritonavir (r), administrée de façon journalière 5 jours par semaine;
- puis un traitement par une trithérapie associant l'emtricitabine (FTC), le tenofovir (TDF) et la lopinavir (LPV) « boostée » par le ritonavir (r), administrée de façon journalière 4 jours par semaine;
- et enfin un traitement par une trithérapie associant l'emtricitabine (FTC), le tenofovir (TDF) et le darunavir (DRV) « boosté » par le ritonavir (r) 400 mg, administrée de façon journalière 4 jours par semaine, puis 3 jours par semaine.

Durant ce traitement, la charge virale plasmatique, ainsi que le taux de TCD4+ dans le sang ont été mesurés.

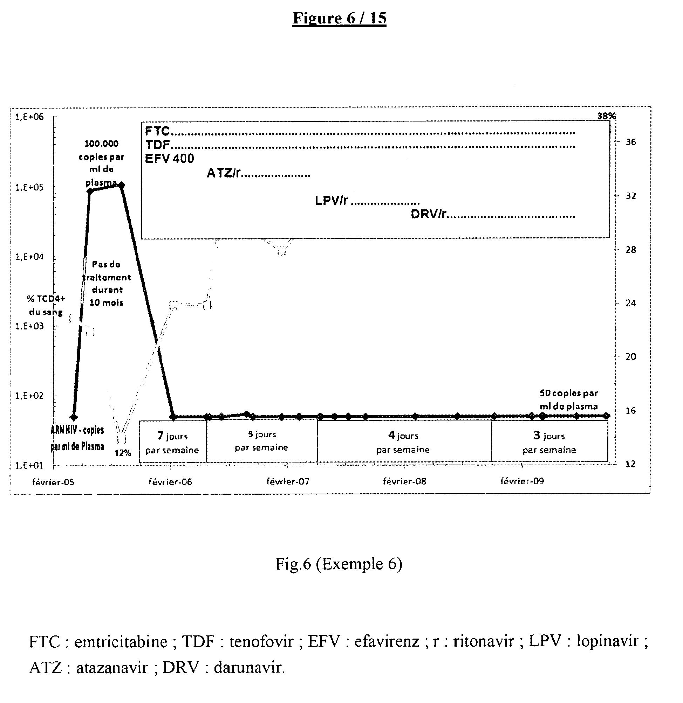

Les résultats sont rapportés dans le Figure 6.

Durant toute la période du traitement, la charge virale plasmatique du patient est demeurée inférieure ou égale à 50 copies/ml de plasma sans qu'aucun échappement virologique ne soit observé. De plus, une remontée du taux de TCD4+ dans le sang a également été observée.

### Exemple 7

Un patient affecté par le VIH a été traité selon le protocole suivant :
- traitement par une quadrithérapie;
- puis un traitement par une trithérapie associant l'emtricitabine (FTC), le tenofovir (TDF) et l'efavirenz 400 mg, administrée de façon journalière 5 jours par semaine, puis 4 jours par semaine, puis 3 jours par semaine.

Durant ce traitement, la charge virale plasmatique, ainsi que le taux de TCD4+ dans le sang ont été mesurés.

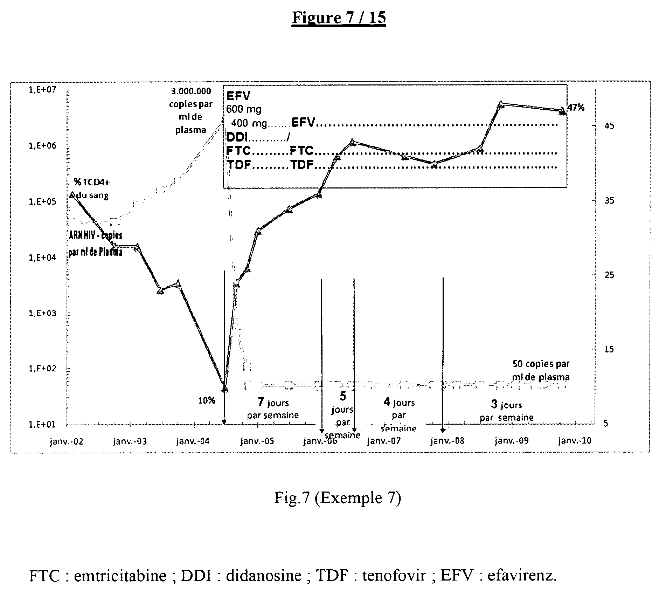

Les résultats sont rapportés dans le Figure 7.

Durant toute la période du traitement, la charge virale plasmatique du patient est demeurée inférieure ou égale à 50 copies/ml de plasma sans qu'aucun échappement virologique ne soit observé. De plus, une remontée du taux de TCD4+ dans le sang a également été observée.

### Exemple 8

Un patient affecté par le VIH a été traité selon le protocole suivant :
- traitement par une quadrithérapie;
- puis un traitement par une trithérapie associant l'emtricitabine (FTC), le tenofovir (TDF) et l'efavirenz, administrée de façon journalière 5 jours par semaine, puis 4 jours par semaine, puis 3 jours par semaine.

Durant ce traitement, la charge virale plasmatique, ainsi que le taux de TCD4+ dans le sang ont été mesurés.

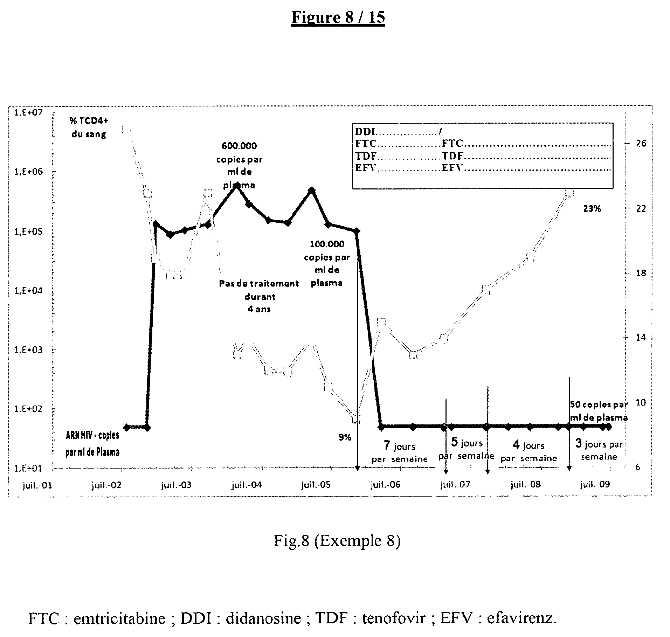

Les résultats sont rapportés dans le Figure 8.

Durant toute la période du traitement, la charge virale plasmatique du patient est demeurée inférieure ou égale à 50 copies/ml de plasma sans qu'aucun échappement virologique ne soit observé. De plus, une remontée du taux de TCD4+ dans le sang a également été observée.

### Exemple 9

Un patient affecté par le VIH a été traité selon le protocole suivant :
- traitement par une quadrithérapie;
- puis un traitement par une trithérapie associant l'emtricitabine (FTC), le tenofovir (TDF) et l'efavirenz 400 mg, administrée de façon journalière 5 jours par semaine, puis 4 jours par semaine.

Durant ce traitement, la charge virale plasmatique, ainsi que le taux de TCD4+ dans le sang ont été mesurés.

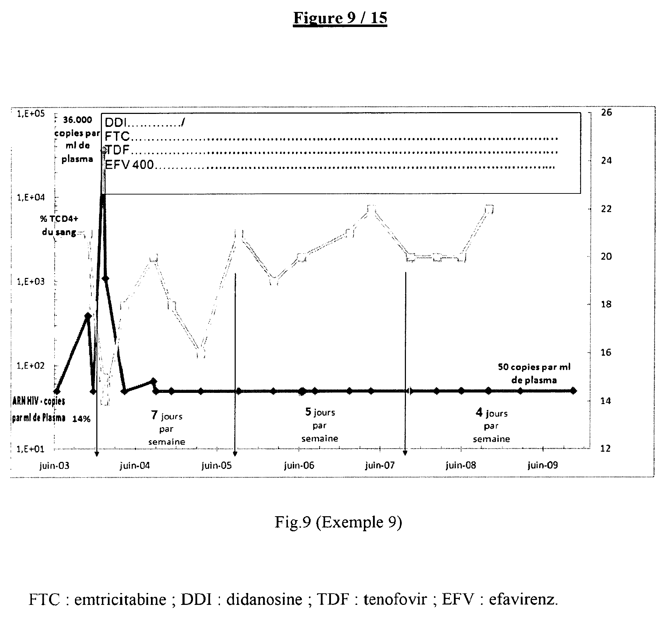

Les résultats sont rapportés dans le Figure 9.

Durant toute la période du traitement, la charge virale plasmatique du patient est demeurée inférieure ou égale à 50 copies/ml de plasma sans qu'aucun échappement virologique ne soit observé. De plus, une remontée du taux de TCD4+ dans le sang a également été observée.

### Exemple 10

Un patient affecté par le VIH non traité durant 80 jours a été traité selon le protocole suivant :
- traitement par une trithérapie associant l'emtricitabine (FTC), le tenofovir (TDF) et l'atazanavir (ATZ) « boostée » par le ritonavir (r) administrée de façon journalière 7 jours par semaine;
- puis un traitement par une trithérapie associant l'emtricitabine (FTC), le tenofovir (TDF) et la lopinavir (LPV) « boostée » par le ritonavir (r), administrée de façon journalière 7 jours par semaine;
- et enfin un traitement par une trithérapie associant l'emtricitabine (FTC), le tenofovir (TDF) et l'efavirenz 400 mg, administrée de façon journalière 5 jours par semaine, puis 4 jours par semaine, puis 3 jours par semaine.

Durant ce traitement, la charge virale plasmatique, ainsi que le taux de TCD4+ dans le sang ont été mesurés.

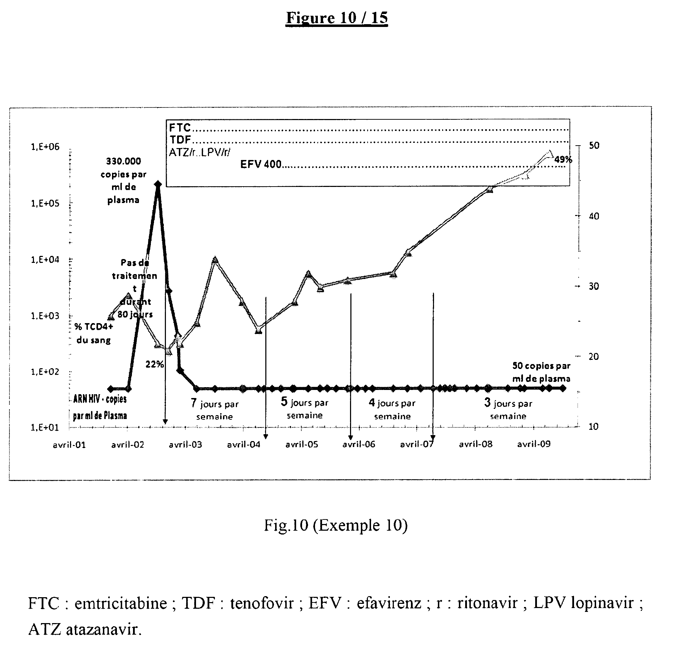

Les résultats sont rapportés dans le Figure 10.

Durant toute la période du traitement, la charge virale plasmatique du patient est demeurée inférieure ou égale à 50 copies/ml de plasma sans qu'aucun échappement virologique ne soit observé. De plus, une remontée du taux de TCD4+ dans le sang a également été observée.

### Exemple 11

Un patient affecté par le VIH a été traité par une trithérapie associant l'emtricitabine (FTC), le tenofovir (TDF) et l'efavirenz, administrée de façon journalière 7 jours par semaine, puis 5 jours par semaine, puis 4 jours par semaine, puis 3 jours par semaine.

Durant ce traitement, la charge virale plasmatique, ainsi que le taux de TCD4+ dans le sang ont été mesurés.

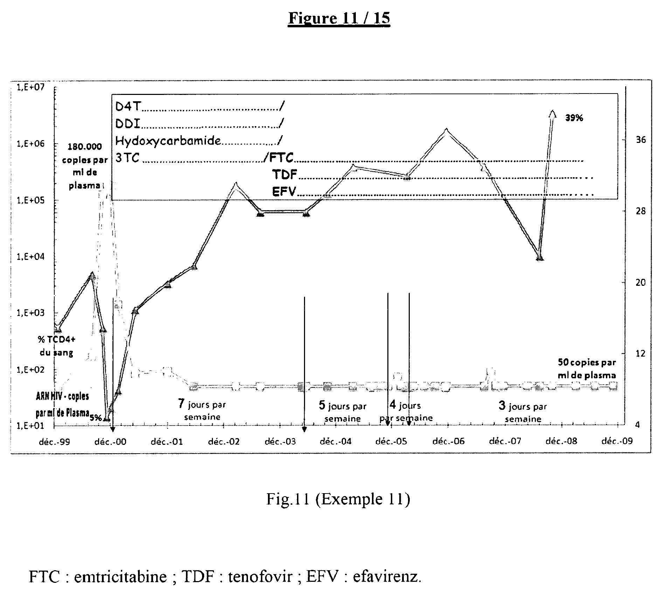

Les résultats sont rapportés dans le Figure 11.

Durant toute la période du traitement, la charge virale plasmatique du patient est demeurée inférieure ou égale à 50 copies/ml de plasma sans qu'aucun échappement virologique ne soit observé. De plus, une remontée du taux de TCD4+ dans le sang a également été observée.

### Exemple 12

Un patient affecté par le VIH a été traité selon le protocole suivant :
- traitement par une quadrithérapie;
- puis un traitement par une trithérapie associant l'emtricitabine (FTC), le tenofovir (TDF) et l'efavirenz 400 mg, administrée de façon journalière 5 jours par semaine, puis 4 jours par semaine, puis 3 jours par semaine.

Durant ce traitement, la charge virale plasmatique, ainsi que le taux de TCD4+ dans le sang ont été mesurés.

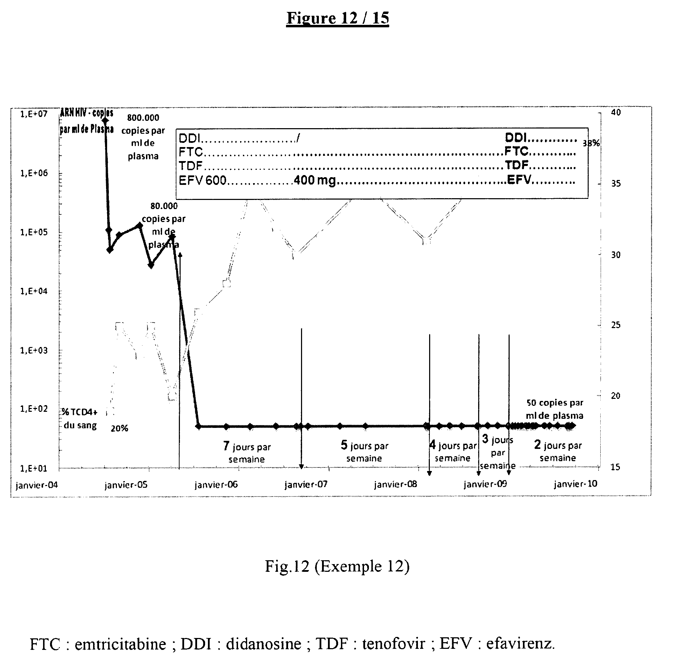

Les résultats sont rapportés dans le Figure 12.

Durant toute la période du traitement, la charge virale plasmatique du patient est demeurée inférieure ou égale à 50 copies/ml de plasma sans qu'aucun échappement virologique ne soit observé. De plus, une remontée du taux de TCD4+ dans le sang a également été observée.

### Exemple 13

Un patient affecté par le VIH a été traité selon le protocole suivant :
- traitement par une quadrithérapie;
- puis un traitement par une trithérapie associant l'emtricitabine (FTC), le tenofovir (TDF) et l'efavirenz 400 mg, administrée de façon journalière 4 jours par semaine.

Durant ce traitement, la charge virale plasmatique, ainsi que le taux de TCD4+ dans le sang ont été mesurés.

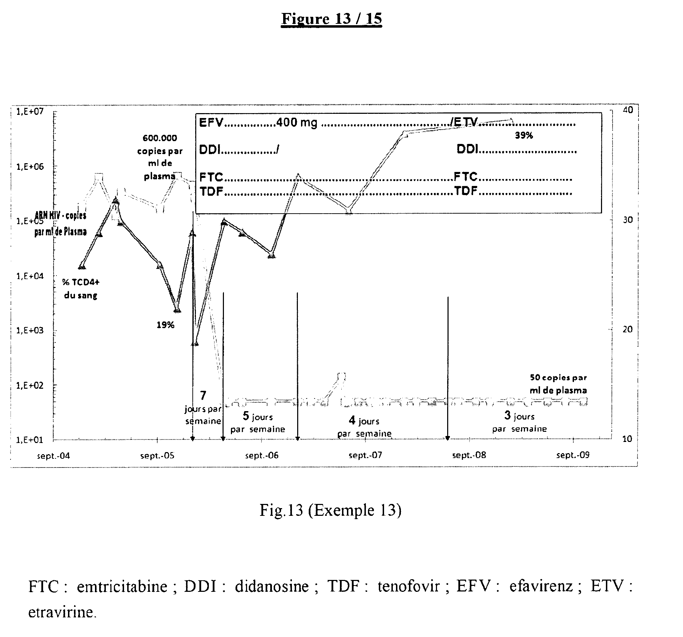

Les résultats sont rapportés dans le Figure 13.

Durant toute la période du traitement, la charge virale plasmatique du patient est demeurée inférieure ou égale à 50 copies/ml de plasma sans qu'aucun échappement virologique ne soit observé. De plus, une remontée du taux de TCD4+ dans le sang a également été observée.

### Exemple 14

Un patient affecté par le VIH a été traité selon le protocole suivant :
- traitement par une quadrithérapie;
- puis un traitement par une trithérapie associant le tenofovir (TDF), l'abacavir et l'efavirenz, administrée de façon journalière 6 jours par semaine, puis 5 jours par semaine, puis 4 jours par semaine, puis 3 jours par semaine.

Durant ce traitement, la charge virale plasmatique, ainsi que le taux de TCD4+ dans le sang ont été mesurés.

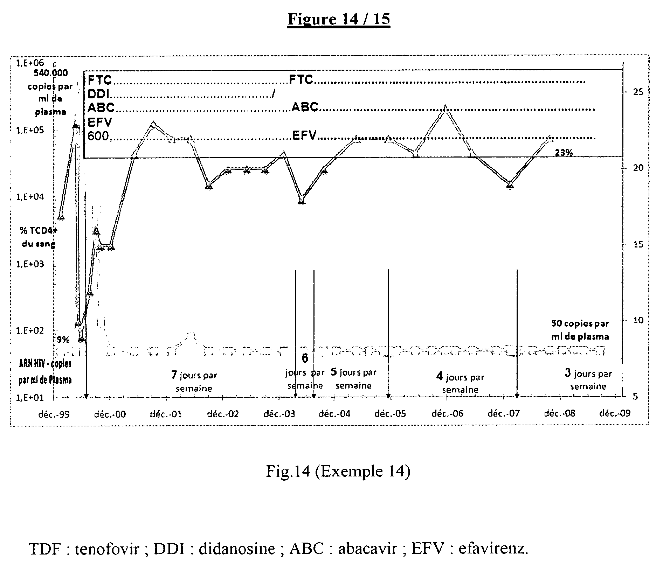

Les résultats sont rapportés dans le Figure 14.

Durant toute la période du traitement, la charge virale plasmatique du patient est demeurée inférieure ou égale à 50 copies/ml de plasma sans qu'aucun échappement virologique ne soit observé. De plus, une remontée du taux de TCD4+ dans le sang a également été observée.

### Exemple 15

Un patient affecté par le VIH a été traité selon le protocole suivant :
- traitement par une quadrithérapie;
- puis un traitement par une trithérapie associant l'emtricitabine (FTC), le tenofovir (TDF) et l'efavirenz 400 mg, administrée de façon journalière 4 jours par semaine.

Durant ce traitement, la charge virale plasmatique, ainsi que le taux de TCD4+ dans le sang ont été mesurés.

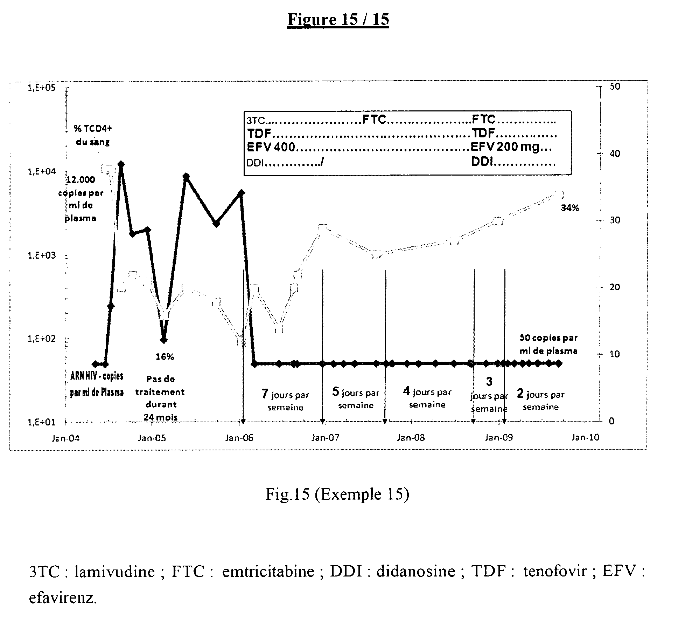

Les résultats sont rapportés dans le Figure 15.

Durant toute la période du traitement, la charge virale plasmatique du patient est demeurée inférieure ou égale à 50 copies/ml de plasma sans qu'aucun échappement virologique ne soit observé. De plus, une remontée du taux de TCD4+ dans le sang a également été observée.

## Revendications

1. Composition pharmaceutique pour le traitement du virus de l'immunodéficience humaine (VIH) chez l'être humain comprenant trois ou quatre principes actifs choisis comme étant :
- un inhibiteur nucléosidique de la transcriptase inverse (INTI) choisi parmi la lamivudine et l'emtricitabine ;
- un autre inhibiteur nucléosidique ou nucléotidique de la transcriptase inverse (INTI) choisi parmi la didanosine, l'abacavir et le tenofovir ; et
- la combinaison du ritonavir avec un inhibiteur de la protéase (IP) choisi parmi le lopinavir, le fosamprenavir, l'atazanavir et le darunavir ; ou un inhibiteur non nucléosidique de la transcriptase inverse (INNTI) choisi parmi l'efavirenz et l'etravirine ;
pour une administration journalière un à quatre jours par semaine audit être humain.

2. Composition pharmaceutique selon la revendication 1, **caractérisée en ce qu'**un INTI est l'emtricitabine.

3. Composition pharmaceutique selon la revendication 1 ou 2, **caractérisée en ce qu'**un INTI est le tenofovir ou la didanosine.

4. Composition pharmaceutique selon l'une quelconque des revendications 1 à 3, **caractérisée en ce qu'**elle comprend la combinaison du ritonavir avec un IP, l'IP étant le darunavir ou l'atazanavir.

5. Composition pharmaceutique selon l'une quelconque des revendications 1 à 4, **caractérisée en ce qu'**elle comprend un INNTI, l'INNTI étant l'efavirenz.

6. Composition pharmaceutique selon l'une quelconque des revendications 1 à 5, **caractérisée en ce qu'**elle comprend, à titre de principes actifs, l'emtricitabine, le tenofovir et l'efavirenz.

7. Composition pharmaceutique selon l'une quelconque des revendications 1 à 6, **caractérisée en ce qu'**elle comprend :
- 300 mg de lamivudine ou 200 mg d'emtricitabine ;
- 250 mg de didanosine, 600 mg d'abacavir ou 245 mg de tenofovir ;
- 100 mg de ritonavir en combinaison avec 600 mg de lopinavir, 1200 mg de fosamprenavir, 300 ou 400 mg d'atazanavir ou 800 ou 900 mg de darunavir ; ou 200, 400 ou 600 mg d'efavirenz ou 400 mg d'etravirine.

8. Composition pharmaceutique selon l'une quelconque des revendications 1 à 7 pour une administration journalière deux à quatre jours par semaine.

9. Composition pharmaceutique selon l'une quelconque des revendications 1 à 7 pour une administration journalière trois ou quatre jours par semaine.

10. Produit pharmaceutique contenant :
- un inhibiteur nucléosidique de la transcriptase inverse (INTI) choisi parmi la lamivudine et l'emtricitabine ;
- un autre inhibiteur nucléosidique ou nucléotidique de la transcriptase inverse (INTI) choisi parmi la didanosine, l'abacavir et le tenofovir ; et
- la combinaison du ritonavir avec un inhibiteur de la protéase (IP) choisi parmi le lopinavir, le fosamprenavir, l'atazanavir et le darunavir ; ou un inhibiteur non nucléosidique de la transcriptase inverse (INNTI) choisi parmi l'efavirenz et l'etravirine ;
comme produit de combinaison pour une administration journalière simultanée, séparée ou étalée dans le temps, un à quatre jours par semaine, dans le cadre du traitement du VIH chez l'être humain.

## Patentansprüche

1. Pharmazeutische Zusammensetzung zur Behandlung des Virus der menschlichen Immundefizienz (VIH) beim Menschen, umfassend drei oder vier Wirkstoffe, ausgewählt aus:
- einem Nukleosidhemmer der inversen Transkriptase (INTI), ausgewählt aus Lamivudin und Emtricitabin;
- einem anderen Nukleosidhemmer oder Nukleotidhemmer der inversen Transkriptase (INTI), ausgewählt aus Didanosin, Abacavir und Tenofovir; und
- der Kombination von Ritonavir mit einem Proteasehemmer (IP), ausgewählt aus Lopinavir, Fosamprenavir, Atazanavir und Darunavir; oder einen Nicht-Nukleosidhemmer der inversen Transkriptase (INNTI), ausgewählt aus Efavirenz und Etravirin;
für eine tägliche Verabreichung an einem bis vier Tagen pro Woche an den Menschen.

2. Pharmazeutische Zusammensetzung nach Anspruch **1, dadurch gekennzeichnet, dass** ein INTI Emtricitabin ist.

3. Pharmazeutische Zusammensetzung nach Anspruch **1** oder **2, dadurch gekennzeichnet, dass** ein INTI Tenofovir oder Didanosin ist.

4. Pharmazeutische Zusammensetzung nach einem der Ansprüche **1** bis **3, dadurch gekennzeichnet, dass** sie die Kombination von Ritonavir mit einem IP umfasst, wobei IP Darunavir oder Atazanavir ist.

5. Pharmazeutische Zusammensetzung nach einem der Ansprüche **1** bis **4, dadurch gekennzeichnet, dass** sie einen INNTI umfasst, wobei der INNTI Efavirenz ist.

6. Pharmazeutische Zusammensetzung nach einem der Ansprüche **1** bis **5, dadurch gekennzeichnet, dass** sie, als Wirkstoffe, Emtricitabin, Tenofovir und Efavirenz umfasst.

7. Pharmazeutische Zusammensetzung nach einem der Ansprüche **1** bis **6, dadurch gekennzeichnet, dass** sie Folgendes umfasst:
- 300 mg Lamivudin oder 200 mg Emtricitabin;
- 250 mg Didanosin, 600 mg Abacavir oder 245 mg Tenofovir;
- 100 mg Ritonavir in Kombination mit 600 mg Lopinavir, 1200 mg Fosamprenavir, 300 oder 400 mg Atazanavir oder 800 oder 900 mg Darunavir; oder 200, 400 oder 600 mg Efavirenz oder 400 mg Etravirin.

8. Pharmazeutische Zusammensetzung nach einem der Ansprüche **1** bis **7** für eine tägliche Verabreichung zwei bis vier Tage pro Woche.

9. Pharmazeutische Zusammensetzung nach einem der Ansprüche **1** bis **7** für eine tägliche Verabreichung drei oder vier Tage pro Woche.

10. Pharmazeutisches Produkt, enthaltend:
- einen Nukleosidhemmer der inversen Transkriptase (INTI), ausgewählt aus Lamivudin und Emtricitabin;
- einen anderen Nukleosidhemmer oder Nukleotidhemmer der inversen Transkriptase (INTI), ausgewählt aus Didanosin, Abacavir und Tenofovir; und
- die Kombination von Ritonavir mit einem Proteasehemmer (IP), ausgewählt aus Lopinavir, Fosamprenavir, Atazanavir und Darunavir; oder einen Nicht-Nukleosidhemmer der inversen Transkriptase (INNTI), ausgewählt aus Efavirenz und Etravirin;
als Kombinationsprodukt für eine gleichzeitige tägliche Verabreichung, getrennt oder zeitlich gestaffelt, einen oder vier Tage pro Woche, im Rahmen der Behandlung von VIH beim Menschen.

## Claims

1. A pharmaceutical composition for treating the human immunodeficiency virus (HIV) in a human being, comprising three or four active principles selected as being:
- one nucleoside inhibitor of reverse transcriptase (NRTI) selected from lamivudine and emtricitabine;
- one nucleoside or nucleotide inhibitors of reverse transcriptase (NRTI) selected from didanosine, abacavir and tenofovir;
- a combination of ritonavir with a protease inhibitor (PI) selected from lopinavir, fosamprenavir, atazanavir and darunavir; or an non-nucleoside reverse transcriptase inhibitor (NNRTI) selected from efavirenz and etravirine;
for daily administration one to four days per week at said human.

2. The pharmaceutical composition according to claim **1,** wherein one of said NRTI is emtricitabine.

3. The pharmaceutical composition according to claims **1** or **2,** wherein one of said NRTI is tenofovir or didanosine.

4. The pharmaceutical composition according to claims **1** to **3,** wherein it comprises the combination of ritonavir with a protease inhibitor (PI), the said PI being atazanavir or darunavir.

5. The pharmaceutical composition according to claims **1** to **4,** wherein it comprises a NNRTI, the NNRTI being efavirenz.

6. The pharmaceutical composition according to claims **1** to **5,** wherein it comprises, as active principles, emtricitabine, tenofovir and efavirenz.

7. The pharmaceutical composition according to claims **1** to **6,** wherein it comprises:
- 300 mg of lamivudine or 200 mg of emtricitabine,
- 250 mg of didanosine, 600 mg of abacavir or 245 mg of tenofovir,
- 100 mg of ritonavir combined with 600 mg of lopinavir, 1200 mg of fosemprenavir, 300 or 400 mg of atazanavir or 800 or 900 mg of darunavir; or 200, 400 or 600 mg of efavirenz or 400 mg of etravirine.

8. The pharmaceutical composition according to claims **1** to **7,** for daily administration two to four days a week.

9. The pharmaceutical composition according to claims **1** to **8,** for daily administration three or four days a week.

10. A pharmaceutical product containing:
- one nucleoside inhibitor of reverse transcriptase (NRTI) selected from lamivudine and emtricitabine;
- one nucleoside or nucleotide inhibitors of reverse transcriptase (NRTI) selected from didanosine, abacavir and tenofovir; and
- a combination of ritonavir with a protease inhibitor (PI) selected from lopinavir, fosamprenavir, atazanavir and darunavir; or an non-nucleoside reverse transcriptase inhibitor (NNRTI) selected from efavirenz and etravirine;
as a combination product for daily simultaneous, separated or spread over time administration one to four days per week, for treating of HIV in a human being.
